# EUROPÄISCHE PATENTANMELDUNG

(11) **EP 1 640 888 A2**
(43) Veröffentlichungstag der Anmeldung: **29.03.2006**
(21) Anmeldenummer: 05019379.6
(22) Anmeldetag: 06.09.2005
(51) Int. Cl.: G06F 19/00

(54) **Verfahren zum Abschätzen und Überwachen des medizinischen Risikos einer Gesundheitsstörung bei einem Patienten**

(30) Priorität: 08.09.2004 DE 102004043312
(71) Anmelder: IpoCare GmbH & Co. KG, 04103 Leipzig (DE)
(72) Erfinder: Löser, Thomas, 04275 Leipzig (DE)
(74) Vertreter: Carlsohn, Alexander

(57) **Zusammenfassung**

Die Erfindung betrifft ein Verfahren zum Abschätzen und Überwachen des medizinischen Risikos einer Gesundheitsstörung bei einem Patienten. Das Verfahren umfaßt
(a) das kontinuierliche Erfassen von Werten von physiologischen Parametern des Patienten, wobei zumindest ein Teil der physiologischen Parameter für das Vorliegen einer Gesundheitsstörung charakteristisch ist;
(b) das Prüfen der Plausibilität jedes erfaßten Wertes der physiologischen Parameter und Abgabe eines Warnsignals, wenn ein erfaßter Wert eines physiologischen Parameters nicht plausibel ist;
(c) das Zuordnen eines Zeitstempels zu jedem erfaßten Wert der physiologischen Parameter;
(d) das Ermitteln von zeitabhängigen Veränderungen der Werte der physiologischen Parameter des Patienten anhand der Zeitstempel;
(e) das Ermitteln eines Risikowertes aus den bis zu einem Zeitpunkt erfaßten Werten der physiologischen Parameter des Patienten und deren zeitabhängigen Veränderungen durch
   Vergleich mit zuvor erfaßten Werten derselben physiologischen Parameter anderer Patienten und deren zeitabhängigen Veränderungen, wobei die zuvor erfaßten Werte und deren zeitabhängige Veränderungen in einer Datenbank gespeichert sind und wobei den zuvor erfaßten Werten der physiologischen Parameter der anderen Patienten und deren zeitabhängigen Veränderungen validierte Risikowerte zugeordnet worden sind, die aus den bei diesem Patienten festgestellten Gesundheitsstörungen ermittelt wurden;
(f) das zuordnen des Risikowertes zu einer Risikoklasse und Ausgabe der Risikoklasse;
(g) das Wiederholen der Schritte (b) bis (g), sobald zumindest ein weiterer Wert eines physiologischen Parameters des Patienten gemäß Schritt (a) erfaßt wird.

## Beschreibung

Die Erfindung betrifft ein Verfahren zum Abschätzen und Überwachen des medizinischen Risikos einer Gesundheitsstörung bei einem Patienten sowie ein Computersystem zur Ausführung dieses Verfahrens.

Im Gesundheitswesen, insbesondere in Kliniken, werden Patientendaten-Managementsysteme eingesetzt, in denen Patientendaten gespeichert werden. Die Patientendaten umfassen neben den sogenannten Stammdaten des Patienten wie Name, Vorname und Geburtsdatum die medizinischen Befunde und eingeleitenden oder bereits abgeschlossenen ärztlichen oder pflegerischen Maßnahmen.

Die medizinischen Befunde sind in der Regel Laborergebnisse, beispielsweise bei Herzerkrankungen relevante Meßwerte wie der Myoglobin-Wert, der Troponin-I-Wert und der CKMB-Wert; bei respiratorischen Erkrankungen relevante Meßwerte wie der Blutgasanalyse, z. B. der pH-Wert des Blutes, der Sauerstoff-Partikaldruck des Blutes, der Kohlendioxid-Partikaldruck des Blutes und die Sauerstoffsättigung des Blutes.

Die in den Patientendaten-Managementsystemen gespeicherten Daten werden durch die den Patienten behandelnden Personen sowie durch das Laborpersonal in das System über standardisierte Schnittstellen (in Krankenhäusern HL7, in Praxen xDT) eingegeben und auf Anforderung dem Behandler zur Verfügung gestellt. Die Daten werden allerdings von dem Patientendaten-Managementsystem nicht analysiert, sondern nur gespeichert und transportiert.

Verfahren zur Analyse von Daten, insbesondere medizinischer Daten sind bekannt. Beispielsweise beschreibt DE 101 45 265 ein Verfahren zur Auswertung von Daten von Patienten mit zumindest einer chronischen Erkrankung, wobei die Patientendaten mit Risikoprofilen verglichen werden, die aus statistischen Daten berechnet worden sind. Anhand der Risikoprofile wird eine Maßnahme für die Behandlung eines Patienten empfohlen. Das Verfahren kann auf einem Computer durchgeführt werden.

DE 694 14 108 offenbart ein System und ein Verfahren zur Bestimmung eines medizinischen Risikos, bei dem die Daten eines Patienten in das System eingegeben werden, aus diesen Daten mittels einer Variablen transformierte Daten errechnet werden und die transformierten Daten mit Mittelwerten und Standardabweichungen aus transformierten Daten anderer Patienten, deren Risiko zuvor mit anderen Systemen bestimmt worden ist, berechnet werden, um die Wahrscheinlichkeit des Auftretens einer Gesundheitsstörung zu berechnen.

Insbesondere bei multimorbiden Patienten ist die Anzahl an Daten, die für jeden einzelnen Patienten durch das Patientendaten-Managementsystem erfaßt werden müssen, außerordentlich hoch. Aufgrund des erheblichen medizinischen Fortschrittes werden inzwischen jedoch bei vermeintlich einfachen Erkrankungen eine Vielzahl von Daten erfaßt, die eine sachgerechte Analyse oft nicht zulassen. Es ist daher nicht untypisch, daß medizinische Entscheidungen anhand einzelner Daten, die als besonders auffallend gelten, getroffen werden. Andere, im Patientendaten-Managementsystem gespeicherte Daten, die einen anderen Befund stützen könnten, werden dabei außer Acht gelassen, so daß aufgrund eher willkürlich ausgewählter Daten Maßnahmen getroffen werden, die sich bei vollständiger Analyse aller Daten als falsch oder unzureichend erweisen könnten. Infolge der angefallenen Datenmenge ist eine solche sachgerechte Analyse jedoch nicht ohne weiteres möglich.

Ferner leiden heutige Patientendaten-Managementsysteme an dem schwerwiegenden Nachteil, daß kritische Zustände von Patienten, die bei vollständiger und verzögerungsfreier Analyse aller Daten des Patienten erkannt werden könnten, nicht oder erst spät festgestellt werden. Dies kann schwerwiegende Konsequenzen für den betroffenen Patienten haben.

Zur Diagnose einer respiratorischen Insuffizienz werden in der heutigen medizinischen Praxis verschiedene Methoden verwendet. Geeignete Methoden sind beispielsweise die Diagnose mittels einer Auskultation, bildgebender Verfahren oder der Blutgasanalyse. Die Blutgasanalyse erfaßt verschiedene physiologische Parameter, die für den Gasgehalt des Blutes und damit die Effizienz der Atmung charakteristisch sind. Allerdings können mittels Auskultation oder bildgebenden Verfahren nur bereits bestehende respiratorische Insuffizienzen erkannt werden; nicht festgestellt werden kann, ob ein erhöhtes Risiko einer solcher Erkrankung besteht oder in naher Zukunft bestehen wird. Eine Blutgasanalyse erfordert eine Analyse einer Vielzahl von Parametern, was - wie vorstehend ausgeführt - Schwierigkeiten bereitet. Im Ergebnis ist es bisher nicht möglich festzustellen, ob ein Risiko einer respiratorischen Insuffizienz besteht oder sich in naher Zukunft entwickeln könnte.

Aufgabe der Erfindung ist es, die Nachteile nach dem Stand der Technik zu beseitigen. Es soll insbesondere ein Verfahren zum Abschätzen und Überwachen des medizinischen Risikos einer Gesundheitsstörung, insbesondere einer respiratorischen Insuffizienz, bei einem Patienten angegeben werden, daß eine frühzeitigere und sichere Abschätzung des Risikos einer Gesundheitsstörung und damit eine sachgerechte medizinische Behandlung ermöglicht, womit eine deutliche Reduzierung der Behandlungszeit eines Patienten verbunden sein kann. Ferner soll ein Computersystem zur Ausführung dieses Verfahrens angegeben werden.

Diese Aufgabe wird durch die Merkmale der Ansprüche 1 und 15 gelöst. Zweckmäßige Ausgestaltungen der Erfindungen ergeben sich aus den Merkmalen der Ansprüche 2 bis 14 sowie 16 und 17.

Nach Maßgabe der Erfindung ist ein Verfahren zum Abschätzen und Überwachen des medizinischen Risikos einer Gesundheitsstörung bei einem Patienten vorgesehen, das folgende Schritte umfaßt:
(a) das kontinuierliche Erfassen von Werten von physiologischen Parametern des Patienten, wobei zumindest ein Teil der physiologischen Parameter für das Vorliegen einer Gesundheitsstörung charakteristisch ist;
(b) das Prüfen der Plausibilität jedes erfaßten Wertes der physiologischen Parameter und Abgabe eines Warnsignals, wenn ein erfaßter Wert eines physiologischen Parameters nicht plausibel ist;
(c) das Zuordnen eines Zeitstempels zu jedem erfaßten Wert der physiologischen Parameter;
(d) das Ermitteln von zeitabhängigen Veränderungen der Werte der physiologischen Parameter des Patienten anhand der Zeitstempel;
(e) das Ermitteln eines Risikowertes aus den bis zu einem Zeitpunkt erfaßten Werten der physiologische Parameter des Patienten und deren zeitabhängiger Veränderungen durch Vergleich mit zuvor erfaßten Werten derselben physiologischen Parameter anderer Patienten und deren zeitabhängigen Veränderungen, wobei die zuvor erfaßten Werten und deren zeitabhängige Veränderungen in einer Datenbank gespeichert sind und wobei den zuvor erfaßten Werte der physiologischen Parameter der anderen Patienten und deren zeitabhängigen Veränderungen validierte Risikowerte zugeordnet worden sind, die aus den bei diesem Patienten festgestellten Gesundheitsstörungen ermittelt wurden;
(f) das Zuordnen des Risikowertes zu einer Risikoklasse und Ausgabe der Risikoklasse; und
(g) das Wiederholen der Schritte (b) bis (g), sobald zumindest ein weiterer Wert eines physiologischen Parameters des Patienten gemäß Schritt (a) erfaßt wird.

Das erfindungsgemäße Verfahren ermittelt einen Risikowert, mit dessen Hilfe das medizinische Risiko einer Gesundheitsstörung früher und sicherer als diese mit herkömmlichen Verfahren möglich ist. Dies ist darauf zurückzuführen, daß im Gegensatz zum Stand der Technik eine Entscheidung über das Risiko einer Gesundheitsstörung nicht anhand eines einzelnen oder einzelner physiologischer Parameter, sondern auf Basis aller Parameter getroffen wird, die für eine Gesundheitsstörung relevant sein könnten und die zueinander in Beziehung gesetzt werden. Ferner ist dies darauf zurückzuführen, das die Werte des Patienten und deren zeitlichen Veränderungen mit Werten und deren zeitliche Veränderungen einer Vielzahl anderer Patienten verglichen werden, die in der Datenbank gespeichert sind. Den Werten und deren zeitliche Veränderungen der anderer Patienten sind validierte Risikowerte zugeordnet. Unter einem validierten Risikowert ist ein Risikowert zu verstehen, der mittels eines anderen Verfahrens als dem erfindungsgemäßen Verfahren gewonnen wurde. Wird das erfindungsgemäße Verfahren beispielsweise zur Bestimmung des Risikos einer respiratorischen Insuffizienz auf Basis physiologischer Parameter, die mittels einer Blutgasanalyse bestimmt werden, verwendet, so kann die Validierung des erfindungsgemäß erhaltenen Risikowertes mittels einer Auskultation erfolgen.

Das Verfahren kann das Auslösen eines optischen und/oder akustischen Alarmes umfassen, wenn der Risikowert einer Risikoklasse zugeordert worden ist, die eine Aktion durch einen Behandler, beispielsweise eine künstliche Beatmung oder Antibiotika-Gaben erfordert.

Die Risikowerte, die erfindungsgemäß aus den erfaßten Werten der physiologischen Parameter des Patienten ermittelt wurden, werden zweckmäßigerweise anhand des tatsächlichen Zustandes des Patienten zum Zeitpunkt der Bestimmung des Risikowertes validiert und gemeinsam mit den Werten der physiologischen Parameter und deren zeitabhängigen Veränderungen in der Datenbank gespeichert, um die Sensitivität und Spezifität des erfindungsgemäßen Verfahrens zu erhöhen.

In einer Ausführungsform der Erfindung sind drei Risikoklassen vorgesehen, wobei die Risikowerte der ersten Risikoklasse zugeordert werden, wenn kein Risiko einer Gesundheitsstörung besteht oder das Risiko einer Gesundheitsstörung abnimmt; die Risikowerte der zweiten Risikoklasse zugeordert werden, wenn sich das Risiko einer Gesundheitsstörung erhöht, ohne daß ein kritischer Zustand des Patienten in einer vorgegebenen Zeiteinheit zu erwarten ist; und die Risikowerte der dritten Risikoklasse zugeordert werden, wenn sich das Risiko einer Gesundheitsstörung derart erhöht, daß ein kritischer Zustand des Patienten in einer vorgegebenen Zeiteinheit zu erwarten ist. Den Risikoklassen können Farben zugeordnet sein, um das Erkennen der Risikoklasse zu erleichtern, beispielsweise wenn diese auf einem Monitor ausgegeben wird. Beispielsweise kann die erste Risikoklasse mit der Farbe Grün hinterlegt werden, die zweite Risikoklasse mit der Farbe Gelb und die dritte Risikoklasse mit der Farbe Rot.

Um die Zuordnung des Risikowertes zu einer Risikoklasse zu erleichtern, wird der ermittelte Risikowert zweckmäßigerweise auf einen Wert zwischen 0 und 1 normiert, wobei "0" kein Risiko einer Gesundheitsstörung und "1" eine Gesundheitsstörung bedeutet. Die erste Risikoklasse könnte dann Risikowerte zwischen 0 und 0,25, die zweite Risikoklasse Risikowerte zwischen 0,25 und 0,45 und die dritte Risikowerte über 0,45 umfassen.

Wird ein Risikowert der dritten Risikoklasse zugeordnet, so wird in einer Ausführungsform der Erfindung ein Alarm, beispielsweise ein optischer und/oder akustischer Alarm, ausgelöst.

Der in Schritt (d) des erfindungsgemäßen Verfahrens vorgenommene Vergleich kann folgendermaßen durchgeführt werden: Aus den zuvor erfaßten Werten der physiologischen Parameter der anderen Patienten, deren zeitabhängige Veränderungen sowie den für die Werte und zeitabhängigen Veränderungen ermittelten validierten Risikowerten wird ein mathematisches Modell des Krankheitsverlaufes berechnet, mit dessen Hilfe plausiblen Werten physiologischer Parameter und deren zeitabhängigen Veränderungen ein Risikowert zugeordnet werden kann. Anschließend wird den erfaßten Werte der physiologischen Parameter des Patienten und deren zeitlichen Veränderungen mittels des mathematischen Modells ein Risikowert zugeordnet. Das mathematische Modell sollte auf einem mulitparmetrischen Algorithmus beruhen.

Das mathematische Modell kann mittels Fuzzifizierung der erfaßten Werte der physiologische Parameter der anderen Patienten, deren zeitabhängigen Veränderungen sowie den für die Werte und zeitabhängigen Veränderungen ermittelten validierten Risikowerten berechnet werden.

Aus dem Stand der Technik ist bekannt, wie auf Basis der hier relevanten Informationen mathematische Modelle berechnet werden können. Beispielsweise wird in EP 0 922 266 B1 ein Verfahren zur Bestimmung des Wachstums von Krebszellen mittels Fuzzy-Logik beschrieben. In DE 103 17 717 wird die Bildung eines mathematischen Modells mittels eines Klassifizierungssystems beschrieben.

Die Sensitivität und die Spezifität des erfindungsgemäßen Verfahrens sollten regelmäßig überprüft werden. Dazu kann beispielsweise das mathematische Modell anhand des validierten Risikowertes des Patienten überprüft und, wenn der validierte Risikowert nicht dem ermittelten Risikowert entspricht, so angepaßt werden, daß nach der Anpassung für dieselben Werte der physiologischen Parameter und deren zeitlichen Veränderungen der ermittelte Risikowert dem validierten Risikowert entspricht. Auf diese Weise wird ein sich selbst verbesserndes Verfahren erhalten, das eine Abschätzung des medizinischen Risikos mit einer im Lauf der Zeit zunehmenden Sensitivität und Spezifität aufweist.

Die Möglichkeit zur ständigen Anpassung des Verfahrens erlaubt es, Änderungen, die sich beispielsweise aus einer Veränderung der Patientenzusammensetzung ergeben, frühzeitig zu erkennen. Sollten zum Beispiel die Patienten einer Klinik, bei denen eine Blutgasanalyse zur Bestimmung des medizinischen Risikos einer respiratorischen Insuffizienz durchgeführt wurde, bisher ausschließlich über 55-jährige Patienten umfassen und sollte die Klinik nunmehr auch jüngere Patienten aufnehmen, bei denen das Risiko einer respiratorischen Insuffizienz besteht, das mittels Blutgasanalyse bestimmt werden soll, so wird dies zunächst mit einer Abnahme der Sensitivität und der Spezifität des erfindungsgemäßen Verfahrens verbunden sein, da mit dem Alter der Patienten sich ein wesentlicher physiologischer Parameter der Patienten insgesamt verändert hat. Durch die Fähigkeit zur Selbstanpassung kann das Verfahren auf diese veränderte Situation früher als bisherige Systeme reagieren, da durch Abnahme der Sensitivität und der Spezifität des erfindungsgemäßen Verfahrens einerseits signalisiert wird, daß eine Veränderung im Hinblick auf die bisherigen Verfahrensbedingungen (zu denen neben dem Alter auch andere Umstände gehören wie beispielsweise eine Veränderung der Meßmethodik der Parameter, die Zusammensetzung der Patienten hinsichtlich Geschlecht oder Risikogruppe, neue medizinische Erkenntnisse im Hinblick auf die Bewertung eines oder mehrerer physiologischen Parameters oder die Hinzunahme weiterer physiologischer Parameter, die bisher aufgrund fehlender medizinischer Kenntnis nicht berücksichtigt wurden, gehören) aufgetreten ist und andererseits durch die Möglichkeit, die Datenbank mit Werten und zugehörigen validierten Risikowerten laufend zu ergänzen, parallel wieder eine Erhöhung der Sensitivität und Spezifität erreicht wird.

Unter dem Begriff "physiologischer Parameter" ist ein Wert zu verstehen, der (a) den Meßwert einer gemessenen physiologischen Kenngröße eines Patienten oder eines Indikatorstoffes angibt (z.B. den Sauerstoffpartialdruck bei einer Blutgasanalyse, Atemfrequenz, Körpertemperatur, CKMB-Konzentration); und (b) eine Klassifizierung eines physiologischen Zustandes eines Patienten (z. B. gelbe Färbung der Gesichtshaut) umfassen kann.

Der Ausdruck "Indikatorstoff" bezieht sich hierin auf Verbindungen oder Elemente, die - je nach ihrer Art - in biologischen Systemen produziert werden oder in biologischen Systeme eingebracht werden und deren Vorhandensein oder deren Konzentration (z. B. in einem bestimmten Organ) ein Charakteristikum für einen biologischen Prozeß oder einen biologischen Zustand ist. Derartige Verbindungen und Elemente umfassen beispielsweise solche, die von Tumorzellen produziert, durch einen Tumor in anderen Körperzellen induziert und/oder als tumorspezifische Stoffe in ihrer Konzentration durch einen Tumor verändert werden. Derartige Indikatorstoffe sind beispielsweise Makromoleküle, z. B. Proteine, oder Spurenelemente. Derartige Verbindungen und Elemente umfassen weiterhin Bonemarker, die für Knochenabbauprozesse wie Osteoporose charakteristisch sind.

Der Begriff "Wert" bezieht sich hier auf alle im medizinischen Bereich (a) anfallende Zahlenwerte für einen physiologischen Parameter (z. B. einen Sauerstoffpartialdruck bei einer Blutgasanalyse in Höhe von 81,2 mmHg, eine Atemfrequenz von 15 Atemzügen pro Minute, eine Körpertemperatur von 26,8 °C, CKMB-Konzentration von 152 ng/ml) oder (b) auf Klassifizierungsergebnisse eines physiologischen Zustandes eines Patienten (z. B. gelbe Färbung der Gesichtshaut: nein, wobei Klassifizierungen mittels Aussagen wie "Nein" ein Zahlenwert, beispielsweise "0" zugeordnet werden sollte).

Vorzugsweise werden zumindest die Werte eines der physiologischen Parameter, die für das Vorliegen einer Gesundheitsstörung charakteristisch sind, unter Verwendung eines Indikatorstoffes bestimmt.

Zur Abschätzung des medizinischen Risikos einer Gesundheitsstörung sollten physiologische Parameter ausgewählt werden, von denen aufgrund medizinischer Erkenntnisse angenommen wird, daß sie im Zusammenhang mit einer bestimmten Gesundheitsstörung, beispielsweise einer respiratorischen Insuffizienz stehen. Zur Bestimmung des medizinischen Risikos einer respiratorischen Insuffizienz werden zum Beispiel physiologische Parameter eingesetzt, die mittels einer Blutgasanalyse gewonnen werden. Die mittels der Blutgasanalyse gewonnenen physiologischen Parameter können den pH-Wert des Blutes, den Sauerstoff-Partialdruck des Blutes, den Kohlendioxid-Partialdruck des Blutes und die Sauerstoffsättigung des Blutes umfassen. Weitere physiologische Parameter, die neben den aus der Blutgasanalyse stammenden Werten zur Abschätzung des medizinischen Risikos einer respiratorischen Insuffizienz in dem erfindungsgemäßen Verfahren verwendet können, umfassen die Atemfrequenz (AF) des Patienten sowie das Alter und das Geschlecht des Patienten.

Das erfindungsgemäße Verfahren kann zur Abschätzung des Risikos jeder Gesundheitsstörung eingesetzt werden, die anhand physiologischer Parameter festgestellt werden kann. Insbesondere kann das Verfahren zur Abschätzung des Risikos des Versagens eines oder mehrerer fester Organe verwendet werden, beispielsweise des Versagens der Lunge, der Niere, der Leber des Herzens oder mehrerer dieser Organe; oder zur Abschätzung des Risikos von Durchblutungsstörungen oder Blutgerinnungsstörungen; oder des Risikos des Auftretens mehrerer dieser Erkrankungen.

Die Zahl der unterschiedlicher physiologischen Parameter, deren Werte zu einem Zeitpunkt in Schritt (d) verarbeitet werden, sollte mindestens 2 betragen.

Unter dem Begriff "kontinuierliches Erfassen" der physiologischen Parameter ist die Erfassung dieser Parameter, sobald diese angefallen sind, zu verstehen.

Zur Prüfung der Plausibilität können Vorgaben, beispielsweise Grenzwerte, für den Wert eines physiologischen Parameters vorgesehen sein. Ferner können solche Vorgaben die Prüfung von Maßeinheiten und Formaten ermöglichen. Überdies sollte die Vollständigkeit der erfaßten physiologischen Parameter geprüft werden, um einen Vergleich mit den Werten der Datenbank zu ermöglichen. Ergibt die Prüfung des erfaßten Wertes, daß dieser Wert den Vorgaben nicht genügt, d. h. unplausibel ist, so erhält der Anwender des erfindungsgemäßen Verfahrens einen technischen Alarm, der ihn z. B. auf eine mögliche Fehleingabe hinweist. Ein solcher Alarm kann in einem optischen Signal bestehen, daß auf einem Monitor dargestellt wird. Der Behandler kann dann entscheiden, ob dieser Wert korrigiert werden muß oder den weiteren Verfahrensschritten unterzogen wird. Entscheidet der Behandler, daß der als unplausibel erkannte Werte weiterverwendet werden soll, so werden in einer bevorzugten Ausführungsform der Erfindung die Vorgaben, die der Plausibilitätsprüfung zugrunde liegen, automatisch so angepaßt, daß zukünftig derartige Werte als plausibel gelten.

Das erfindungsgemäße Verfahren ist nicht auf die Ausgabe von Risikowerten beschränkt. Vielmehr können anhand des zeitlichen Verlaufs der Risikowerte durch Vergleich mit den validierten Risikowerten und deren zeitlichen Verläufen eine Prognose über die zukünftige Entwicklung des medizinischen Risikos einer Gesundheitsstörung ermittelt werden. Ferner können die Risikowerte mit Diagnosevorschlägen, Vorschlägen zur Verbesserung des Meßregimes und Therapiepfaden verknüpft werden.

Nachfolgend wird die Erfindung nachstehend anhand von Zeichnungen näher erläutet. Dabei zeigt
- Fig. 1: eine schematische Darstellung einer Ausführungsform des Verfahrens;
- Fig. 2: eine schematische Darstellung einer Ausführungsform des erfindungsgemäßen Computersystems, und
- Fig. 3: eine Darstellung eines Monitorbildes, das den ermittelten Risikowert ausgibt.

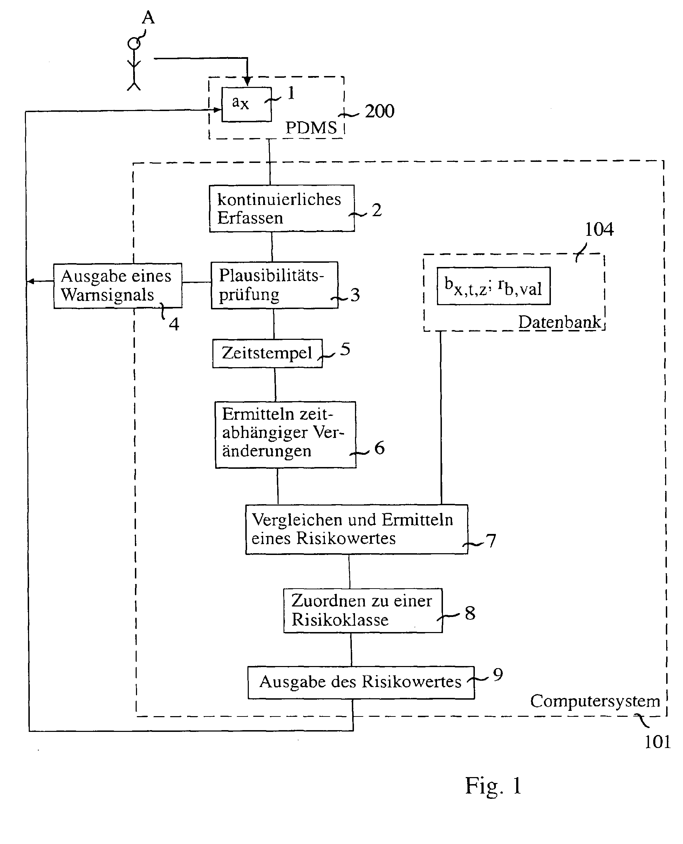

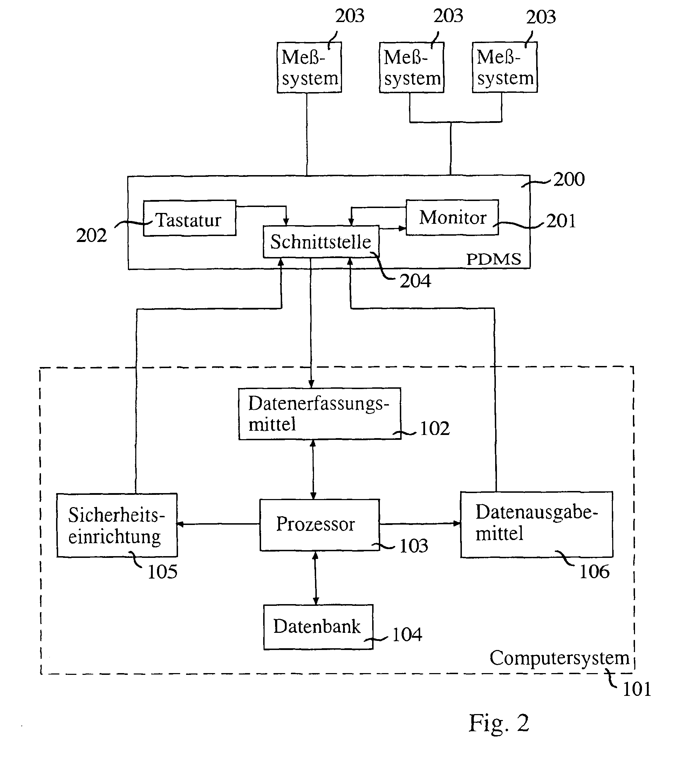

Nach Fig. 1 werden Werte aₓ (x = 1 ... n, wobei n für die Gesamtzahl aller physiologischen Parameter, die für den Patienten A erfaßt worden sind, steht; x den jeweiligen physiologischen Parameter kennzeichnet) ausgewählter physiologischer Parameter x eines Patienten A kontinuierlich in einem Computersystem 101 (siehe Fig. 2) erfaßt 2. Das Computersystem 101 ist über eine standardisierte Schnittstelle 204 wie HL7 mit einem Patientendatenmanagementsystem 200 verbunden, von dem es die Werte der physiologischen Parameter aₓ über die Datenerfassungsmittel 102 erhält. Die physiologischen Parameter aₓ können im Patientendatenmanagementsystem 200 mittels eines Terminals über eine Tastatur 202 eingegeben oder direkt von den Meßgeräten 203, die die physiologischen Parameter aₓ messen, übernommen werden.

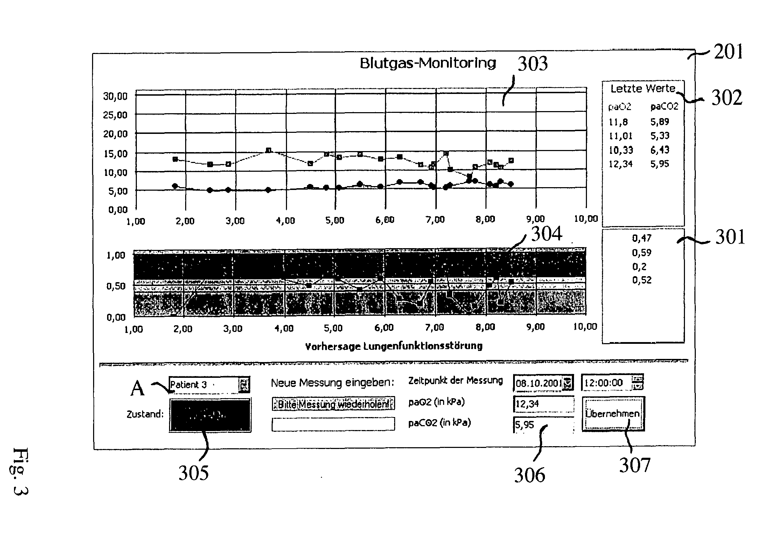

Nach der Erfassung 2 der physiologischen Parameter aₓ werden die erfaßten Werte hinsichtlich ihrer Plausibilität geprüft 3. Genügen die Werte nicht den Vorgaben, so wird ein Warnsignals abgegeben 4, das auf einem Monitor 201 eines Patientendatenmanagementsystem 200 dargestellt werden kann (siehe Fig. 3).

Im Anschluß oder vor der Plausibilitätsprüfung wird jedem erfaßten Wert der physiologischen Parameter aₓ ein Zeitstempel zugeordnet 5, so daß die Werte a_{x,t} erhalten werden, wobei t die Zeit kennzeichnet, die dem Wert aₓ zugeordnet worden ist. Auf Basis der Zeitstempel werden zeitabhängige Veränderungen Δa der Werte der physiologischen Parameter a_{x,t} des Patienten A ermittelt 6. Durch Vergleich 7 der bis zu einem Zeitpunkt erfaßten Werte der physiologischen Parameter a_{x,t} des Patienten A und deren zeitabhängiger Veränderungen Δa durch Vergleich mit zuvor erfaßten Werten b_{x,t.z} (wobei x dieselbe Bedeutung wie oben hat, t die dem Wert zugeordnete Zeit in bezug auf den Krankheitsverlauf des Patienten z ist und z eine Laufzahl für jeden anderen Patienten als A ist) derselben physiologischen Parameter x anderer Patienten z und deren zeitabhängigen Veränderungen Δb wird ein Risikowert r_{A,t} berechnet, der das zu diesem Zeitpunkt bestehende Risiko einer Gesundheitsstörung kennzeichnet. Die Werte bx, t, z und deren zeitabhängige Veränderungen Δb sind in einer Datenbank 104 gespeichert, wobei den Werten bx, t, z der physiologischen Parametern x der anderen Patienten z und deren zeitabhängigen Veränderungen Δb validierte Risikowerte r_{b,val} zugeordnet worden sind, die aus den bei den Patienten z festgestellten Gesundheitsstörungen ermittelt wurden.

Anschließend wird der so erhaltene Risikowert r_{A,t} zu einer Risikoklasse Rₘ (wobei m für die Zahl der Klassen steht) zugeordnet 8, und der Risikowert r und die Risikoklasse werden über die Datenausgabemittel 106 an das Patientendatenmanagementsystem 200 ausgegeben 9 und dort auf dem Monitor 201 (siehe Fig. 3). Die Risikoklasse ist farblich hinterlegt.

Die Plausibilitätsprüfung 3, die Zuordnung 5 des Zeitstempels, die Ermittlung 6 zeitabhängiger Veränderungen, der Vergleich 7 und die Zuordnung 8 wird vorzugsweise mittels eines Prozessors, der in dem Computersystem 101 vorgesehen ist, vorgenommen .

Sobald zumindest ein neuer Wert aₓ für einen physiologischen Parameter vorliegt, wird ein neuer Risikowert r_{a,t} berechnet. Die Risikowerte, die zu unterschiedlichen Zeitpunkten erhalten werden, können ebenfalls auf dem Monitor 201 des Patientendatenmanagementsystems 200 dargestellt werden.

Das Computersystem 101 weist eine Sicherheitseinrichtung 105 auf, die in vorgegebenen zeitlichen Abständen ein Signal ("Lebenszeichen") an das Patientendatenmanagementsystem 200 sendet. Sollte zu einem vorgegebenen Zeitpunkt kein Signal eingehen, so wird ein Alarm ausgeben und, falls erwünscht, automatisch versucht, die Verbindung zwischen Patientendaten-managementsystem 200 und dem Computersystem 101 wieder herzustellen.

Nach Fig. 3 können die Werte a_{x,t} und die ermittelten Risikowerte r_{a,t} in Form eines Diagramms dargestellt werden. Ferner können weitere Daten, die eine Entscheidung über die im Hinblick auf das zutreffende Risiko erleichtern, auf dem Monitor 201 dargestellt werden.

Auf dem Monitor 201 werden Daten ausgegeben, die im Zusammenhang mit einer Blutgasanalyse stehen. Es werden die Risikowerte r_{a,t} 301 eines Patienten A dargestellt, wobei der jüngste Risikowerte der oberste Werte ist. Die Risikowerte 301 sind überdies in einem Diagramm 304 dargestellt, dessen Abszisse die Zeit t zeigt, die dem Wert 301 zugeordnet worden ist, und dessen Ordinate die Risikowerte 301 zeigt. Ferner sind die letzten Werte aₓ (Bezugszeichen 302) der gemessenen physiologischen Parameter x dargestellt. Folgende physiologische Parameter wurden erfaßt: der Sauerstoff-Partialdruck paO2 und Kohlendioxid-Partialdruck paCO2. Die jüngsten Werte sind die obersten Werte. Ferner werden die Werte 302 in einem Diagramm 303 dargestellt, dessen Abszisse die Zeit t zeigt, die dem Wert 302 zugeordnet worden ist, und dessen Ordinate die Werte 302 darstellt.

Der letzte Risikowert 301 ist einer Risikoklasse zugeordnet, die mittels einer Farbe 305 signalisiert wird. Ferner ist jede Risikoklasse mit einem Erläuterungstext (hier "Auffällig") versehen, der das Risiko einer Gesundheitsstörung prägnant beschreibt.

Neue Werte 306 der beiden physiologischen Parameter paO2 und paCO2 können mit einer Tastatur 202 eingegeben werden. Die Werte 306 werden ebenfalls auf dem Monitor 201 angezeigt. Deren Verarbeitung mittels des erfindungsgemäßen Verfahrens kann mit einer Taste 307 gestartet werden.

Nachfolgend wird die Erfindung nachstehend anhand eines Beispiels weiter erläutert.

### Beispiel: Blutgasanalyse

Bei 47 neurochirurgisch versorgten und beatmeten Patienten wurde das Risiko einer respiratorischen Insuffizienz mittels des erfindungsgemäßen Verfahrens auf Basis von Blutgasanalysen sowie der Atemfrequenz-Werte, des Alters und des Geschlechtes des Patienten bestimmt. Die Blutgasanalyse (BSA) umfaßt die Bestimmung von Werten für folgende physiologische Parameter: pH-Wert des Blutes, Sauerstoff-Partikaldruck des Blutes, Kohlendioxid-Partikaldruck des Blutes und Sauerstoffsättigung des Blutes. Insgesamt wurden 757 BSA-Messungen duchgeführt.

Mittels des erfindungsgemäßen Verfahrens konnte bei 31 eine respiratorische Insuffizienz mit einer Sensitivität von 84 % und einer Spezifität von 91,5 % 26 h früher gegenüber herkömmlichen Verfahren (Auskultation) erkannt werden, so daß frühzeitig geeignete Behandlungsmaßnahmen ergriffen wurden. Das hatte eine Verminderung der Liegezeit zur Folge. Bei 16 Patienten wurde keine respiratorische Insuffizienz festgestellt. Der Vergleich gemäß Schritt (d) wurde unter Verwendung eines mathematisches Modell auf Basis eines regelbasierten und/oder musterbasierten multiparametrischen Fuzzy-Algorithmus durchgeführt.

Bezugszeichenliste
- 1: Werte aₓ physiologischer Parameter des Patienten A
- 2: Erfassen der Werte aₓ von dem Patientendatenmanagementsystem (PDMS) 200
- 3: Prüfen der Werte aₓ auf Plausibilität
- 4: Abgabe eines Warnsignals
- 5: Zuordnen eines Zeitstempels zu jedem Wert aₓ unter Erhalt der Werte a_{x,t}
- 6: Ermitteln von zeitabhängigen Veränderungen Δa zwischen Werten a_{x,t} für unterschiedliche Zeitpunkte t
- 7: Vergleich mit den Werten a_{x,t,z} einer Datenbank und Ermitteln eines Riskowertes r_{A,t}
- 8: Zuordnen eines Risikowertes r_{A,t} zu einer Risikoklasse
- 9: Ausgabe des Risikowertes r_{A,t} und der Risikoklasse an das Patientendatenmanagementsystem 200

- 101: Computersystem
- 102: Datenerfassungsmittel
- 103: Prozessor
- 104: Datenbank
- 105: Sicherheitseinrichtung
- 106: Datenausgabemittel

- 200: Patientendatenmanagementsystem
- 201: Monitor
- 202: Tastatur
- 203: Meßsystem
- 204: Schnittstelle

- 301: Risikowerte
- 302: Werte a von paO2 und paCO2
- 303: Diagramm der Werte a in bezug auf die Zeit t
- 304: Diagramm der Risikowerte in bezug auf die Zeit t
- 305: Farbliche Darstellung der Risikoklasse
- 306: Neue Werte a von pa02 und paCO2, die bisher nicht zur Abschätzung des Risikos verwendet wurden
- 307: Taste, um das Erfassen der Werte 306 durch das erfindungsgemäße Verfahren auszulösen

## Patentansprüche

1. Verfahren zum Abschätzen und Überwachen des medizinischen Risikos einer Gesundheitsstörung bei einem Patienten, umfassend
(a) das kontinuierliche Erfassen von Werten von physiologischen Parametern des Patienten, wobei zumindest ein Teil der physiologischen Parameter für das Vorliegen einer Gesundheitsstörung charakteristisch ist;
(b) das Prüfen der Plausibilität jedes erfaßten Wertes der physiologischen Parameter und Abgabe eines Warnsignals, wenn ein erfaßter Wert eines physiologischen Parameters nicht plausibel ist;
(c) das Zuordnen eines Zeitstempels zu jedem erfaßten Wert der physiologischen Parameter;
(d) das Ermitteln von zeitabhängigen Veränderungen der Werte der physiologischen Parameter des Patienten anhand der Zeitstempel;
(e) das Ermitteln eines Risikowertes aus den bis zu einem Zeitpunkt erfaßten Werten der physiologischen Parameter des Patienten und deren zeitabhängigen Veränderungen durch
Vergleich mit zuvor erfaßten Werten derselben physiologischen Parameter anderer Patienten und deren zeitabhängigen Veränderungen, wobei die zuvor erfaßten Werte und deren zeitabhängige Veränderungen in einer Datenbank gespeichert sind und wobei den zuvor erfaßten Werten der physiologischen Parametern der anderen Patienten und deren zeitabhängigen Veränderungen validierte Risikowerte zugeordnet worden sind, die aus den bei diesem Patienten festgestellten Gesundheitsstörungen ermittelt wurden;
(f) das Zuordnen des Risikowertes zu einer Risikoklasse und Ausgabe der Risikoklasse;
(g) das Wiederholen der Schritte (b) bis (g), sobald zumindest ein weiterer Wert eines physiologischen Parameters des Patienten gemäß Schritt (a) erfaßt wird.

2. Verfahren nach Anspruch 1, **dadurch gekennzeichnet, daß** es ferner das Auslösen eines optischen und/oder akustischen Alarmes umfaßt, wenn der Risikowert einer Risikoklasse zugeordert worden ist, die eine medizinische Handlung erfordert.

3. Verfahren nach Anspruch 1 oder Anspruch 2, **dadurch gekennzeichnet, daß** die Risikowerte, die aus den erfaßten Werten der physiologischen Parameter des Patienten ermittelt wurden, anhand des tatsächlichen Zustandes des Patienten zum Zeitpunkt der Bestimmung des Risikowertes validiert werden.

4. Verfahren nach einem der vorstehen Ansprüche, **dadurch gekennzeichnet, daß** die Werte der physiologischen Parameter des Patienten und die validierten Risikowerte für diese Werte in der Datenbank gespeichert werden.

5. Verfahren nach einem der vorstehenden Ansprüche, **dadurch gekennzeichnet, daß** drei Risikoklassen vorgesehen sind, wobei die Risikowerte der ersten Risikoklasse zugeordert werden, wenn kein Risiko einer Gesundheitsstörung besteht oder das Risiko einer Gesundheitsstörung abnimmt; die Risikowerte der zweiten Risikoklasse zugeordert werden, wenn sich das Risiko einer Gesundheitsstörung erhöht, ohne daß ein kritischer Zustand des Patienten in einer vorgegebenen Zeiteinheit zu erwarten ist; und die Risikowerte der dritten Risikoklasse zugeordert werden, wenn sich das Risiko einer Gesundheitsstörung derart erhöht, daß ein kritischer Zustand des Patienten in einer vorgegebenen Zeiteinheit zu erwarten ist.

6. Verfahren nach Anspruch 5, **dadurch gekennzeichnet, daß** ein Alarm ausgelöst wird, wenn der Risikowert der dritten Risikoklasse zugeordnet wird.

7. Verfahren nach einem der vorstehenden Ansprüche, **dadurch gekennzeichnet, daß** zur Durchführung des Vergleiches in Schritt (d)
(d1) aus den zuvor erfaßten Werten der physiologischen Parameter der anderen Patienten, deren zeitabhängigen Veränderungen sowie den für die Werte und zeitabhängigen Veränderungen ermittelten validierten Risikowerten ein mathematisches Modell des Krankheitsverlaufes berechnet wird, mit dessen Hilfe plausiblen Werten physiologischer Parameter und deren zeitabhängigen Veränderungen ein Risikowert zugeordnet werden kann; und
(d2) den erfaßten Werten der physiologischen Parameter des Patienten und deren zeitlichen Veränderungen mittels des mathematischen Modells ein Risikowert zugeordnet wird.

8. Verfahren nach Anspruch 7, **dadurch gekennzeichnet, daß** das mathematische Modell mit Fuzzifizierung der erfaßten Werte der physiologischen Parameter der anderen Patienten, deren zeitabhängigen Veränderungen sowie den für die Werte und zeitabhängigen Veränderungen ermittelten validierten Risikowerten berechnet wird.

9. Verfahren nach Anspruch 7 oder Anspruch 8, **dadurch gekennzeichnet, daß** das mathematische Modell anhand des validierten Risikowertes des Patienten überprüft und, wenn der validierte Risikowert nicht dem ermittelten Risikowert entspricht, so angepaßt wird, daß nach der Anpassung für dieselben Werte der physiologischen Parameter und deren zeitabhängigen Veränderungen der ermittelte Risikowert dem validierten Risikowert entspricht.

10. Verfahren nach einem der vorstehenden Ansprüche, **dadurch gekennzeichnet, daß** zumindest die Werte eines der physiologischen Parameter, die für das Vorliegen einer Gesundheitsstörung charakteristisch sind, unter Verwendung eines Indikatorstoffes bestimmt werden.

11. Verfahren nach einem der vorstehenden Ansprüche, **dadurch gekennzeichnet, daß** die physiologischen Parameter mittels einer Blutgasanalyse gewonnen werden.

12. Verfahren nach Anspruch 11, **dadurch gekennzeichnet, daß** die mittels der Blutgasanalyse bestimmten physiologischen Parameter den pH-Wert des Blutes, den Sauerstoff-Partialdruck des Blutes, den Kohlendioxid-Partialdruck des Blutes und die Sauerstoffsättigung des Blutes umfassen.

13. Verfahren nach Anspruch 12, **dadurch gekennzeichnet, daß** die physiologischen Parameter ferner die Atemfrequenz umfassen.

14. Verfahren nach einem der vorstehenden Ansprüche, **dadurch gekennzeichnet, daß** die physiologischen Parameter das Alter oder das Geschlecht des Patienten umfassen.

15. Computersystem zum Abschätzen und Überwachen des medizinischen Risikos einer Gesundheitsstörung bei einem Patienten, umfassend
- Datenerfassungsmittel (102) zum kontinuierlichen Erfassen von Werten von physiologischen Parametern des Patienten, wobei zumindest ein Teil der physiologischen Parameter für das Vorliegen einer Gesundheitsstörung charakteristisch ist;
- Mittel (104) zum Speichern einer Datenbank;
- Mittel (103) zum Prüfen der Plausibilität jedes erfaßten Wertes der physiologischen Parameter; zum Zuordnen eines Zeitstempels zu jedem erfaßten Wert der physiologischen Parameter; zum Ermitteln von zeitabhängigen Veränderungen der Werte der physiologischen Parameter des Patienten anhand der Zeitstempel; zum Ermitteln eines Risikowertes aus den bis zu einem Zeitpunkt erfaßten Werten der physiologischen Parameter des Patienten und deren zeitabhängigen Veränderungen durch Vergleich mit zuvor erfaßten Werten derselben physiologischen Parameter anderer Patienten und deren zeitabhängigen Veränderungen, wobei die zuvor erfaßten Werte und deren zeitabhängige Veränderungen in der Datenbank gespeichert sind und wobei den zuvor erfaßten Werten der physiologischen Parameter der anderen Patienten und deren zeitabhängigen Veränderungen validierte Risikowerte zugeordnet worden sind, die aus den bei diesem Patienten festgestellten Gesundheitsstörungen ermittelt wurden; und zum Zuordnen des Risikowertes zu einer Risikoklasse; und
- Datenausgabemittel (106) zur Ausgabe eines Risikowertes und einer Risikoklasse.

16. Computersystem nach Anspruch 15, **dadurch gekennzeichnet, daß** es ferner ein Alarmierungsmittel zur Ausgabe eines optischen/und akustischen Alarms, wenn ein erfaßter Wert eines physiologischen Parameters nicht plausibel ist und/oder wenn der Risikowert einer Risikoklasse zugeordnet worden ist, bei der ein kritischer Zustand des Patienten in einer vorgegebenen Zeiteinheit zu erwarten ist, umfaßt.

17. Computersystem nach Anspruch 15 oder Anspruch 16, **dadurch gekennzeichnet, daß** die Datenerfassungsmittel (102) und die Datenausgabemittel (106) über eine standardisierte Schnittstelle (204) mit einem Patientendatenmanagementsystem (200) verbunden sind.
